# EUROPÄISCHE PATENTSCHRIFT

(11) **EP 0 485 742 B1**
(45) Veröffentlichungstag und Bekanntmachung des Hinweises auf die Patenterteilung: **29.03.1995**
(21) Anmeldenummer: 91117317.7
(22) Anmeldetag: 10.10.1991
(51) Int. Cl.: A61B 6/06

(54) **Primärstrahlenblende für eine Röntgenröhre**
X-Ray primary radiation diaphragm assembly
Diaphragme pour le rayonnement X primaire

(30) Priorität: 14.11.1990 SE 9003638
(43) Veröffentlichungstag der Anmeldung: 20.05.1992
(73) Patentinhaber: Siemens-Elema AB, 171 95 Solna 1 (SE); SIEMENS AKTIENGESELLSCHAFT, 80333 München (DE)
(72) Erfinder: Warden, Hans SE, S-194 51 Upplads Väsby (SE)

(56) Entgegenhaltungen:
- DE-A- 1 441 312
- FR-A- 635 489
- GB-A- 832 373
- US-A- 1 909 118

## Beschreibung

Die Erfindung betrifft eine Primärstrahlenblende für eine Röntgenröhre mit einer ersten Gruppe von zwei senkrecht zueinander nahe dem Fokus der Röntgenröhre angeordneten Blendenplattenpaaren sowie mit einer zweiten Gruppe von zwei senkrecht zueinander angeordneten fokusfernen Blendenplattenpaaren, wobei die Blendenplatten jedes Blendenplattenpaares mit Hilfe von Steuermitteln in Bezug auf die Längsachse der Blende gemeinsam einstellbar sind und wobei die erste Gruppe von Blendenplattenpaaren und die zweite Gruppe von Blendenplattenpaaren in gleicher Weise symmetrisch gegen die Längsachse gerichtet sind und mittels-Einstellmitteln derart verstellbar sind, daß eine Strahlentrahlenpyramide gewünschter Größe gebildet wird, deren Spitze im Fokus liegt und die durch die erste und die zweite Gruppe von Blendenplattenpaaren begrenzt ist.

Eine Primärstrahlenblende dieser Art ist durch die DE-OS 1 441 312 bekannt. Die Einstellung der Blendenplatten erfolgt mittels Antriebsrollen und Antriebsriemen, die die Primärstrahlenblende in ihrem Aufbau verhältnismäßig kompliziert macht. Außerdem kann wegen des Riemenantriebs bei der Verschiebung der Blendenplatten ein gewisses Spiel entstehen, was die Genauigkeit bei der Einstellung der Primärstrahlenblende negativ beeinflußt.

Der Erfindung liegt die Aufgabe zugrunde, eine Primärstrahlenblende der eingangs genannten Art zu schaffen, die im Aufbau einfach ist und trotzdem bei der Einstellung der Blendenplatten eine hohe Präzision aufweist.

Diese Aufgabe ist erfindungsgemäß dadurch gelöst, daß die Einstellmittel eine einzige erste Welle umfassen, die das Steuermittel eines Blendenplattenpaares in der ersten Gruppe mit dem steuermittel eines hierzu parallel angeordneten Blendenplattenpaares in der zweiten Gruppe direkt verbindet, und eine weitere einzige Welle, die das Steuermittel des Blendenplattenpaares in der ersten Gruppe mit dem Steuermittel des übrigen Blendenplattenpaares in der zweiten Gruppe direkt verbindet. Hierdurch ist ein sehr einfacher Aufbau einer Primärstrahlenblende der genannten Art gegeben, die aufgrund der direkten Verbindung der Wellen zwischen den steuermitteln der Blendenplattenpaare eine sehr hohe Präzision bei der Einstellung der Blendenplatten aufweist. Wegen des Aufbaus der Primärstrahlenblende ist es auch verhältnismäßig einfach, diese mit weiteren Blendplatten zu ergänzen, die zwischen der ersten und der zweiten Gruppe der Blendenplattenpaare angebracht werden können. Die Steuermittel der weiteren Blendenplattenpaare werden dann mit den Wellen verbunden, die auf diese Weise gemeinsam sämtliche Blendenplattenpaare steuern. Durch diese weiteren Blendenplatten ist eine wirkungsvollere Ausblendung der Röntgenstrahlen möglich.

Eine alternative Lösung wird in Anspruch 7 definiert. Eine Primärstrahlblende nach dem Oberbegriff des Anspruchs 7 ist aus FR-A-635 489 bekannt.

In einer vorteilhaften Ausbildung der Erfindung wird vorgeschlagen, daß die Blendenplatten jedes Blendenplattenpaares Teile eines Parallelogrammarmsystems sind und daß das Steuermittel des Blendenplattenpaares ein Zahnsegment und ein Zahnrad aufweist, das in das Zahnsegment eingreift, wobei das Zahnsegment direkt an einem Arm im Parallelogrammarmsystem angebracht ist. Hierdurch ist eine sehr genaue Übertragung der Bewegung des Zahnrades bzw. Zahnsegments auf die Blendenplattenpaare gegeben.

In einer Weiterbildung der Erfindung wird vorgeschlagen, daß die Blendenplatten jedes Blendenplattenpaares Teile eines Parallelogrammarmsystems sind und daß das Steuermittel des Blendenplattenpaares ein Zahnsegment und ein Zahnrad aufweist, das im Zahnsegment eingreift, wobei das Zahnsegment selbst einen Arm im Parallelogrammarmsystem bildet. Hierdurch wird erreicht, daß das Parallelogrammarmsystem verhältnismäßig klein gemacht werden kann, was auch bedeutet, daß die Blendenplatten entsprechend klein sind. Ein derartiges Blendenplattenpaar ist geeignet, in die Nähe des Fokuses der Röntgenröhre gebracht zu werden, da es die Spitze der Strahlungspyramide bilden kann.

Eine konstruktiv einfache Ausführung der Erfindung ist dadurch gegeben, daß die Zahnräder an den Wellen fest angebracht sind.

Im Hinblick auf eine vorteilhafte Weiterbildung der Erfindung wird vorgeschlagen, daß die erste Gruppe von Blendenplattenpaaren größenmäßig kleiner ist als die zweite Gruppe von Blendenplattenpaaren, so daß die Primärstrahlenblende die Form eines Kegelstumpfes annimmt. Hierdurch kann die Primärstrahlenblende verhältnismäßig klein und leicht gemacht werden, was insbesonders bei der Verwendung in Verbindung mit einem Eintank für einen mobilen Röntgengenerator vorteilhaft ist, da der Eintank so klein und leicht wie möglich gehalten werden soll.

In einer weiteren konstruktiv einfachen Ausführung der Erfindung wird vorgeschlagen, daß die Zähne der Zahnsegmente entsprechend den Winkeln der Wellen im Verhältnis zur Längsachse der Blende geformt sind. Hierdurch ist erreicht, daß die Zahnräder, die an den Wellen angebracht sind, unabhängig von den Winkeln der Wellen immer gerade sind.

Die Erfindung bezieht sich auch auf eine Primärstrahlenblende für eine Röntgenröhre mit einer ersten Gruppe von zwei senkrecht zueinander angeordneten Blendenplattenpaare, wobei die Blendenplatten jedes Blendenplattenpaares Teile jeweils eines Parallelogrammarmsystems sind und im Verhältnis zur Längsachse der Blende mit Hilfe jeweils eines Zahnsegments gemeinsam einstellbar sind, wobei jedes Zahnsegment mit Hilfe eines jeweiligen Zahnrades um eine Achse drehbar ist.

Eine Primärstrahlenblende dieser Art ist durch das Siemens Datenblatt "RUREK X-ray Collimator" bekannt. Diese Blende ist eine Tiefenblende mit zwei Blendenebenen, bei dem die zweite Blende eine Irisblende ist. Die Einstellung dieser Blendenebenen erfolgt getrennt.

Eine Vereinfachung und eine Verbesserung der Präzision bei der Einstellung einer Zweiebenenblende der genannten Art wird erreicht, indem die Primärstrahlenblende eine zweite Gruppe von zwei senkrecht zueinander angeordneten Blendenplattenpaaren umfaßt, die in ihrem Aufbau und Einstellbarmöglichkeiten der ersten Gruppe von Blendenplattenpaaren entsprechen und die mit Abstand von der ersten Gruppe von Blendenplattenpaaren in Längsrichtung der Längsachse gesehen angebracht sind, wobei die erste Gruppe von Blendenplattenpaaren und die zweite Gruppe von Blendenplattenpaaren in gleicher Weise symmetrisch gegen die Längsachse gerichtet sind und wobei ein Zahnrad für die Einstellung eines Blendenplattenpaares in der ersten Gruppe und ein entsprechendes Zahnrad für die Einstellung eines Blendenplattenpaares in der zweiten Gruppe auf einer gemeinsamen drehbaren Welle angebracht sind.

In einer Weiterentwicklung dieser Ausführungsform der Erfindung wird vorgeschlagen, daß das übrige Zahnrad für die erste Gruppe von Blendenplattenpaaren und das übrige Zahnrad für die zweite Gruppe von Blendenplattenpaaren auf einer zweiten gemeinsamen drehbaren Welle fest angebracht sind. Auf diese Weise wird erreicht, daß auch die restlichen Blendenplattenpaare der Primärstrahlenblende dieselbe Einstellungsmöglichkeiten aufweisen wie die weiteren Blendenplattenpaare.

Weitere Vorteile und Einzelheiten ergeben sich aus den Unteransprüchen.

Die Erfindung ist nachfolgend anhand eines in den Zeichnungen dargestellten Ausführungsbeispiels näher erläutert.
Es zeigen:
FIG. 1 eine Seitenansicht einer Primärstrahlenblende nach der Erfindung,
FIG. 2 eine Draufsicht einer Primärstrahlenblende nach FIG 1 und
FIG. 3 ein Schnitt durch die Primärstrahlenblende nach der Schnittlinie III-III in der FIG. 1.

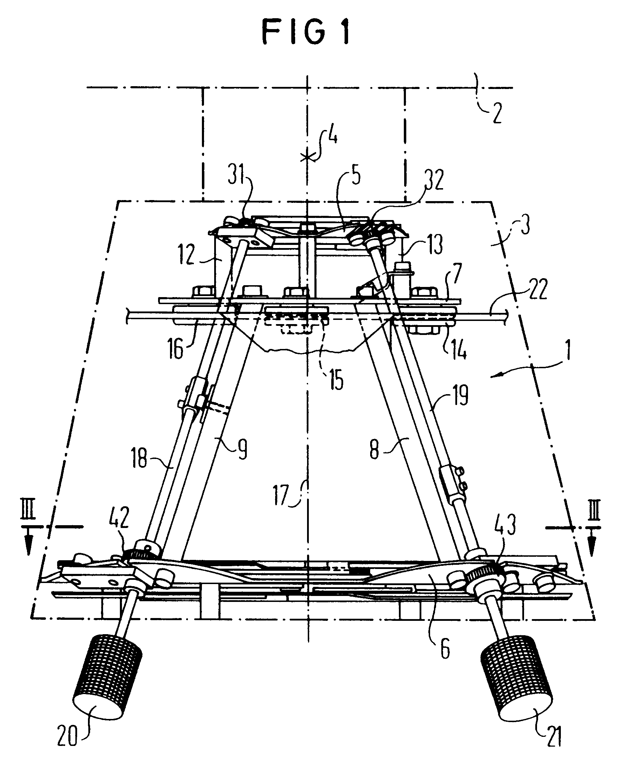

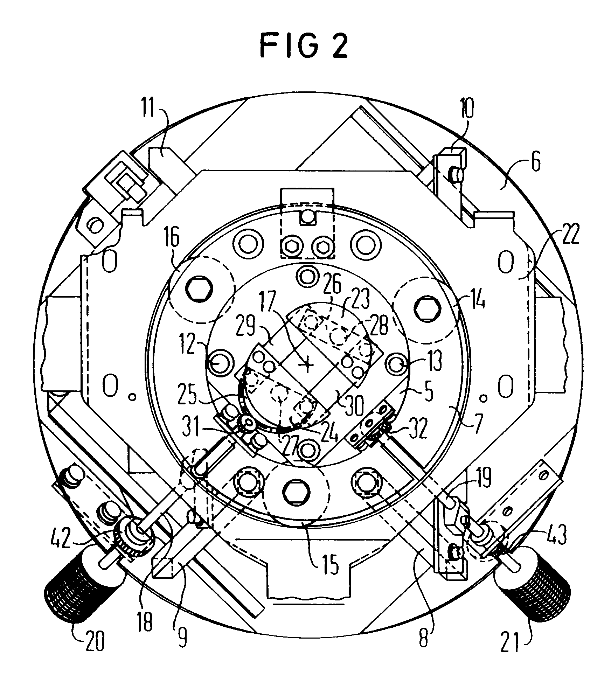

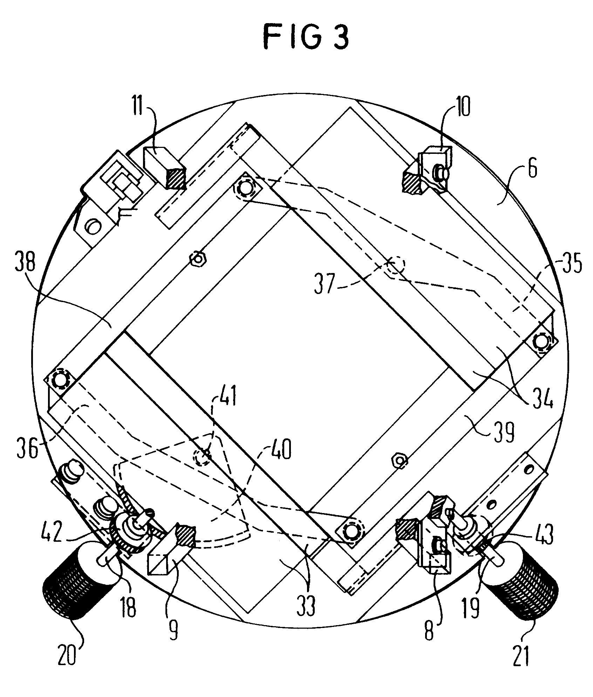

In der FIG. 1 ist eine Primärstrahlenblende 1 für eine Röntgenröhre 2 gezeigt, die in einem Gehäuse 3 angebracht ist. Die Blende 1 ist eine Tiefenblende mit zwei Blendenebenen mit einer ersten Gruppe von zwei senkrecht zueinander angeordneten Blendenplattenpaaren, die nahe am Fokus 4 der Röntgenröhre 1 und im Anschluß an einen Aufsatz 5 angebracht sind. Diese Blendenplattenpaare werden in Verbindung mit der FIG. 2 näher beschrieben. Eine zweite Gruppe von zwei senkrecht zueinander angeordneten Blendenplattenpaaren, die vom Fokus 4 weiter entfernt liegen, sind im Anschluß an einen weiteren Aufsatz 6 angebracht. Die zuletzt erwähnten Blendenplattenpaare werden in Verbindung mit der FIG. 3 näher beschrieben. Aus FIG. 1 geht hervor, daß der Aufsatz 5 mit der ersten Gruppe von Blendenplattenpaare kleiner ist als der Aufsatz 6 mit der zweiten Gruppe von Blendenplattenpaare, so daß die Primärstrahlenblende 1 die Form eines Kegelstumpfes annimmt. Zwischen den Aufsätzen 5 und 6 ist eine Platte 7 angebracht. Zwischen dieser Platte 7 und dem Aufsatz 6 sind Distanzelemente 8 bis 11 vorgesehen, wobei in der Figur lediglich die Distanzelemente 8 und 9 gezeigt werden. Zwischen der Platte 7 und dem Aufsatz 5 sind weitere Distanzelemente 12 und 13 befestigt. Die Platte 7 trägt Rollen 14,15 und 16 mit Nuten, in denen ein Führungsblech 22, das im Gehäuse 3 befestigt ist, angeordnet ist. Durch diesen Aufbau kann die Primärstrahlenblende um ihre Längsachse 17 gedreht werden.

Von den Gruppen von Blendenplattenpaaren, die an dem Aufsatz 5 befestigt sind, ist das eine Blendenplattenpaar an der oberen Seite und das andere Blendenplattenpaar an der unteren Seite des Aufsatzes 5 befestigt. Die weitere Gruppe von Blendenplattenpaaren ist so verteilt, daß das eine Blendenplattenpaar an der oberen Seite und das andere Blendenplattenpaar an der unteren Seite des Aufsatzes 6 befestigt ist. Die Blendenplattenpaare, die an der oberen Seite der Aufsätze 5,6 angebracht sind, sind über später näher beschriebene Steuermittel, die aus Zahnrädern und Zahnsegmenten bestehen, mittels einer Welle 18 miteinander verbunden. Die Blendenplattenpaare, die an der unteren Seite der Aufsätze 5,6 angebracht sind, sind auch über später beschriebene Steuermittel, die aus Zahnrädern und Zahnsegmenten bestehen, mittels einer Welle 19 miteinander verbunden. Jede Welle 18,19 weist einen Drehknopf 20,21 auf. Durch Drehen der Drehknöpfe 20,21 werden die Wellen 18,19 um ihre jeweiligen Längsachsen gedreht, wobei die Blendenplattenpaare, die mit den jeweiligen Wellen 18,19 verbunden sind, in eine gewünschte Lage eingestellt werden können.

In der FIG. 2 werden Blendenplattenpaare 23,24 gezeigt, die an der Oberseite des Aufsatzes 5 angebracht sind. Aus der Figur geht auch hervor, daß die Blendenplatten 23,24 Teile eines Parallelogrammarmsystems sind, bestehend aus Querarmen 25,26, die um jeweils eine Achse 27,28 drehbar sind, und aus Längsarmen 29,30, die um die Querarme 25,26 drehbar befestigt sind. Die eine Blendenplatte 24 ist an dem Arm 29 und die andere Blendenplatte 23 ist an dem Arm 30 befestigt. In dem gezeigten Parallelogrammarmsystem ist der Querarm 25 ein Zahnsegment. Das Zahnsegment 25 ist um die Achse 27 mit Hilfe eines Zahnrades 31 drehbar, das an der Welle 18 befestigt ist und gegen das Zahnsegment 25 anliegt. Durch Drehen des Zahnsegments 25 mit Hilfe des Zahnrades 31 werden die Blendenplatten 23,24 gegeneinander verschoben, bis eine gewünschte Position erreicht ist. Dasjenige Blendenplattenpaar, das senkrecht zu dem zuerst genannten Blendenplattenpaar 23,24 angebracht ist und welches an der unteren Seite des Aufsatzes 5 befestigt und daher in der FIG. 2 nicht gezeigt ist, unterscheidet sich im Aufbau nicht von dem beschriebenen Blendenplattenpaar 23,24, außer daß das Zahnsegment für die Steuerung der nicht gezeigten Blendenplatten mit Hilfe eines an der Welle 19 befestigten Zahnrades 32 gedreht wird. Durch Drehen des Zahnsegments mittels des Zahnrades 32 werden auch diese Blendenplatten gegeneinander in eine gewünschte Lage gegeneinander verschoben.

In der FIG. 3 wird dasjenige Blendenplattenpaar 33,34 gezeigt, das an der oberen Seite des Aufsatzes 6 angebracht ist. Die Blendenplatten 33,34 sind an einem Parallelogrammarmsystem mit Querarmen 35,36 befestigt, wobei der Querarm 35 um eine Welle 37 drehbar ist. Das Parallelogrammarmsystem weist außerdem Längsarme 38,39 auf, die um die Querarme 35,36 drehbar sind. Dabei ist die Blendenplatte 33 am Arm 38 und die Blendenplatte 34 am Arm 39 befestigt. Ein Zahnsegment 40 ist direkt am Querarm 36 angebracht und um eine Achse 41 drehbar angeordnet. Das Zahnsegment 40 und somit auch der Arm 36 können nun um die Achse 41 mit Hilfe eines Zahnrades 40, das an der Welle 18 befestigt ist und im Zahnsegment eingreift, gedreht werden. Durch Drehen des Zahnsegments 40 werden die Blendenplatten 33,34 gegeneinander in eine gewünschte Position verschoben. Dasjenige Blendenplattenpaar, das senkrecht zu dem zuletzt genannten Blendenplattenpaar 33,34 an der unteren Seite des Aufsatzes 6 angebracht und daher in der FIG. 3 nicht dargestellt ist, unterscheidet sich im Aufbau nicht von dem Blendenplattenpaar 33,34, außer daß das Zahnsegment für die Steuerung der nicht gezeigten Blendenplatten mit Hilfe eines an der Welle 19 befestigten Zahnrades 43 gedreht wird. Durch Drehen des Zahnsegments werden die Blendenplatten gegeneinander in eine gewünschte Position verschoben.

Die Zähne sämtlicher Zahnsegmente sind nach den Winkeln der Wellen 18,19 im Verhältnis zur Längsachse 17 der Blende geformt. Auf diese Weise werden lediglich gerade Zahnräder verwendet, die an den Wellen 18,19 befestigt sind.

Um das Blendenplattenpaar 23,24 an der oberen Seite des Aufsatzes 5 und das Blendenplattenpaar 33,34 an der Oberseite des Aufsatzes 6 in eine gewünschte Position zu bringen, wird lediglich an dem Drehknopf 20 gedreht, bis die Blendenplatten diese Position erreicht haben. In der gleichen Weise werden auch die unterhalb den Aufsätzen 5,6 angebrachten Blendenplatten, die gegenüber den Blendenplatten 23,24 und 33,34 um 90°verschoben sind, mit Hilfe des Drehknopfes 21 gesteuert.

Die Übersetzung zwischen den Zahnrädern bzw. den Zahnsegmenten am Aufsatz 5 und den Zahnräden bzw. den Zahnsegmenten am Aufsatz 6 ist so gewählt, daß die Blendenplattenpaare am Aufsatz 6 in einfacher Weise eingestellt werden können, so daß bei einer eingeschalteten Blende eine Pyramide gewünschter Größe gebildet werden kann.

## Patentansprüche

1. Primärstrahlenblende für eine Röntgenröhre mit einer ersten Gruppe von zwei senkrecht zueinander nahe dem Fokus der Röntgenröhre angeordneten Blendenplattenpaaren sowie mit einer zweiten Gruppe von zwei senkrecht zueinander angeordneten fokusfernen Blendenplattenpaaren, wobei die Blendenplatten jedes Blendenplattenpaares mit Hilfe von Steuermitteln in Bezug auf die Längsachse der Blende gemeinsam einstellbar sind und wobei die erste Gruppe von Blendenplattenpaaren und die zweite Gruppe von Blendenplattenpaaren in gleicher Weise symmetrisch gegen die Längsachse gerichtet und mittels Einstellmitteln derart verstellbar sind, daß eine Strahlenpyramide gewünschter Größe gebildet wird, deren Spitze im Fokus liegt und die durch die erste und die zweite Gruppe von Blendenplattenpaaren begrenzt ist, **dadurch gekennzeichnet,** daß die Einstellmittel eine einzelne erste Welle (18) umfassen, die das Steuermittel (25,31) eines Blendenplattenpaares (23,24) in der ersten Gruppe mit dem Steuermittel (40,42) eines hierzu parallel angeordneten Blendenplattenpaares (33,34) in der zweiten Gruppe direkt verbindet, und eine weitere einzelne Welle (19), die das Steuermittel (32) des übrigen Blendenplattenpaares in der ersten Gruppe mit dem Steuermittel (43) des übrigen Blendenplattenpaares in der zweiten Gruppe direkt verbindet.

2. Primärstrahlenblende nach Anspruch 1, **dadurch gekennzeichnet**, daß die Blendenplatten (33,34) jedes Blendenplattenpaares Teile eines Parallelogrammarmsystems sind und daß das Steuermittel des Blendenplattenpaares (33,34) ein Zahnsegment (40), und ein Zahnrad (42) aufweist, das in das Zahnsegment (40) eingreift, wobei das Zahnsegment (40) direkt an einem Arm (36) im Parallelogramarmsystem angebracht ist.

3. Primärstrahlenblende nach Anspruch 1, **dadurch gekennzeichnet,** daß die Blendenplatten (23,24) jedes Blendenplattenpaares Teile eines Parallelogrammarmsystems sind und daß das Steuermittel des Blendenplattenpaares (23,24) ein Zahnsegment (25) und ein Zahnrad (31) aufweist, das in das Zahnsegment (25) eingreift, wobei das Zahnsegment (25) selbst einen Arm im Parallelogrammarmsystem bildet.

4. Primärstrahlenblende nach einem der Ansprüche 1 bis 3, **dadurch gekennzeichnet,** daß die Zahnräder (31,32,42,43) an den Wellen (18,19) fest angebracht sind.

5. Primärstrahlenblende nach einem der Ansprüche 1 bis 4, **dadurch gekennzeichnet,** daß die erste Gruppe von Blendenplattenpaaren (23,24) größenmäßig kleiner ist als die zweite Gruppe von Blendenplattenpaaren (33,34), so daß die Primärstrahlenblende (1) die Form eines Kegelstumpfes annimmt.

6. Primärstrahlenblende nach einem der Ansprüche 1 bis 5. **dadurch gekennzeichnet,** daß die Zähne der Zahnsegmente (25,40) entsprechend den Winkeln der Wellen (18,19) im Verhältnis zur Längsachse (17) der Blende (1) geformt sind.

7. Primärstrahlenblende für eine Röntgenröhre mit einer ersten Gruppe von zwei senkrecht zueinander angeordneten Blendenplattenpaaren, wobei die Blendenplatten jedes Blendenplattenpaares Teile jeweils eines Parallelogrammarmsystems sind und im Verhältnis zur Längsachse der Blende mit Hilfe jeweils eines Zahnsegments gemeinsam einstellbar sind, wobei jedes Zahnsegment mit Hilfe eines jeweiligen Zahnrades um eine Achse drehbar ist, **dadurch** **gekennzeichnet**, daß die Primärstrahlenblende eine zweite Gruppe von zwei senkrecht zueinander angeordneten Blendenplattenpaaren (33,34) aufweist, die in ihrem Aufbau und Einstellmöglichkeiten der ersten Gruppe von Blendenplattenpaaren (23,24) entspricht und die mit Abstand von der ersten Gruppe von Blendenplattenpaaren (23,24), in der Längsrichtung der Längsachse (17) gesehen, angebracht ist, wobei die erste Gruppe von Blendenplattenpaaren (23,24) und die zweite Gruppe von Blendenplattenpaaren (33,34) in gleicher Weise symmetrisch gegen die Längsachse (17) gerichtet sind und wobei ein Zahnrad (31) für die Einstellung eines Blendenplattenpaares (23,24) in der ersten Gruppe und ein entsprechendes Zahnrad (42) für die Einstellung eines Blendenplattenpaares (33,34) in der zweiten Gruppe auf einer gemeinsamen drehbaren Welle (18) angebracht sind.

8. Primärstrahlenblende nach Anspruch 7, **dadurch gekennzeichnet**, daß das übrige Zahnrad (32) für die erste Gruppe von Blendenplattenpaaren und das übrige Zahnrad (43) für die zweite Gruppe von Blendenplattenpaaren auf einer zweiten gemeinsamen drehbaren Welle (19) fest angebracht sind.

9. Primärstrahlenblende nach Anspruch 7 oder 8, **dadurch gekennzeichnet**, daß die Zahnsegmente (40) direkt an einem Arm (36) im Parallelogramarmsystem angebracht sind.

10. Primärstrahlenblende nach Anspruch 7 oder 8, **dadurch gekennzeichnet**, daß die Zahnsegmente (25) einen Arm im Parallelogrammarmsystem bilden.

11. Primärstrahlenblende nach einem der Ansprüche 7 bis 10 **dadurch gekennzeichnet,** daß die erste Gruppe von Blendenplattenpaaren (23,24) größenmäßig kleiner ist als die zweite Gruppe von Blendenplattenpaaren (33,34), so daß die Primärstrahlenblende (1) die Form eines Kegelstumpfes annimmt.

12. Primärstrahlenblende nach einem der Ansprüche 7 bis 11, **dadurch gekennzeichnet,** daß die Zähne der Zahnsegmente (25,40) entsprechend den Winkeln der Wellen (18,19) im Verhältnis zur Längsachse (17) der Blende (1) geformt sind.

## Claims

1. Primary radiation diaphragm for an X-ray tube having a first group of two pairs of diaphragm plates arranged perpendicular to each other close to the focus of the X-ray tube and having a second group of two pairs of diaphragm plates arranged perpendicular to each other remote from the focus, the diaphragm plates of each pair of diaphragm plates being able to be set together with reference to the longitudinal axis of the diaphragm with the aid of control means, and the first group of pairs of diaphragm plates and the second group of pairs of diaphragm plates being directed in a similar fashion symmetrically towards the longitudinal axis and being adjustable by means of setting means in such a way that a radiation pyramid of desired size is formed, whose point lies in the focus and which is limited by the first and second group of pairs of diaphragm plates, characterized in that the setting means comprise a first individual shaft (18) which directly connects the control means (25, 31) of a pair of diaphragm plates (23, 24) in the first group to the control means (40, 42) of a pair of diaphragm plates (33, 34) arranged parallel hereto in the second group, and a further individual shaft (19) which directly connects the control means (32) of the remaining pair of diaphragm plates in the first group to the control means (43) of the remaining pair of diaphragm plates in the second group.

2. Primary radiation diaphragm according to Claim 1, characterized in that the diaphragm plates (33, 34) of each pair of diaphragm plates are parts of a parallelogram arm system and in that the control means of the pair of diaphragm plates (33, 34) has a toothed segment (40) and a gearwheel (42) which engages in the toothed segment (40), the toothed segment (40) being fitted directly on one arm (36) in the parallelogram arm system.

3. Primary radiation diaphragm according to Claim 1, characterized in that the diaphragm plates (23, 24) of each pair of diaphragm plates are parts of a parallelogram arm system and in that the control means of the pair of diaphragm plates (23, 24) has a toothed segment (25) and a gearwheel (31) which engages in the toothed segment (25), the toothed segment (25) itself forming one arm in the parallelogram arm system.

4. Primary radiation diaphragm according to one of Claims 1 to 3, characterized in that the gearwheels (31, 32, 42, 43) are fixed permanently on the shafts (18, 19).

5. Primary radiation diaphragm according to one of Claims 1 to 4, characterized in that the first group of pairs of diaphragm plates (23, 24) is smaller in terms of size than the second group of pairs of diaphragm plates (33, 34), so that the primary radiation diaphragm (1) assumes the form of a truncated cone.

6. Primary radiation diaphragm according to one of Claims 1 to 5, characterized in that the teeth of the toothed segments (25, 40) are formed corresponding to the angles of the shafts (18, 19) in relation to the longitudinal axis (17) of the diaphragm (1).

7. Primary radiation diaphragm for an X-ray tube having a first group of two pairs of diaphragm plates arranged perpendicular to each other, the diaphragm plates of each pair of diaphragm plates being respectively parts of a parallelogram arm system and being able to be set together with respect to the longitudinal axis of the diaphragm with the aid in each case of a toothed segment, each toothed segment being rotatable about a pivot with the aid of a respective gearwheel, characterized in that the primary radiation diaphragm has a second group of two pairs of diaphragm plates (33, 34) arranged perpendicular to each other, which group corresponds in its construction and setting capabilities to the first group of pairs of diaphragm plates (23, 24) and is fitted at a distance from the first group of pairs of diaphragm plates (23, 24), seen in the longitudinal direction of the longitudinal axis (17), the first group of pairs of diaphragm plates (23, 24) and the second group of pairs of diaphragm plates (33, 34) being directed in a similar fashion symmetrically towards the longitudinal axis (17), and a gearwheel (31) for the setting of one pair of diaphragm plates (23, 24) in the first group and a corresponding gearwheel (42) for the setting of a pair of diaphragm plates (33, 34) in the second group being fitted on a common rotatable shaft (18).

8. Primary radiation diaphragm according to Claim 7, characterized in that the remaining gearwheel (32) for the first group of pairs of diaphragm plates and the remaining gearwheel (43) for the second group of pairs of diaphragm plates are fitted permanently on a second common rotatable shaft (19).

9. Primary radiation diaphragm according to Claim 7 or 8, characterized in that the toothed segments (40) are fitted directly on one arm (36) in the parallelogram arm system.

10. Primary radiation diaphragm according to Claim 7 or 8, characterized in that the toothed segments (25) form one arm in the parallelogram arm system.

11. Primary radiation diaphragm according to one of Claims 7 to 10, characterized in that the first group of pairs of diaphragm plates (23, 24) is smaller in terms of size than the second group of pairs of diaphragm plates (33, 34), so that the primary radiation diaphragm (1) assumes the form of a truncated cone.

12. Primary radiation diaphragm according to one of Claims 7 to 11, characterized in that the teeth of the toothed segments (25, 40) are formed corresponding to the angles of the shafts (18, 19) in relation to the longitudinal axis (17) of the diaphragm (1).

## Revendications

1. Diaphragme du rayonnement primaire pour un tube à rayons X comportant un premier groupe formé de deux couples de plaques de diaphragme, disposées perpendiculairement l'une par rapport à l'autre à proximité du foyer du tube à rayons X, ainsi qu'un second groupe formé de deux couples de plaques de diaphragme éloignées du foyer et disposées perpendiculairement l'une à l'autre, et dans lequel les plaques de chaque couple de plaques de diaphragme peuvent être réglées en commun par rapport à l'axe longitudinal du diaphragme, à l'aide de moyens de commande et dans lequel le premier groupe de couples de plaques de diaphragme et le second groupe de couples de plaques de diaphragme sont dirigés symétriquement de la même manière par rapport à l'axe longitudinal et sont réglables à l'aide de moyens de réglage de telle sorte qu'il se forme une pyramide de rayonnement de taille désirée, dont la pointe est située au foyer et qui est limitée par les premier et second groupes de couples de plaques de diaphragme, caractérisé par le fait que les moyens de réglage comprennent un premier arbre individuel (18), qui relie directement le moyen de commande (25,31) d'un premier couple (23,24) de plaques de diaphragme du premier groupe au moyen de commande (40,42) d'un couple (33,34) de plaques de diaphragme, parallèles aux précédentes, du second groupe, et un autre arbre individuel (19), qui relie directement le moyen de commande (32) de l'autre couple de plaques de diaphragme du premier groupe au moyen de commande (43) de l'autre couple de plaques de diaphragme du second groupe.

2. Diaphragme du rayonnement primaire suivant la revendication 1, caractérisé par le fait que les plaques (33, 34) de chaque couple de plaques de diaphragme font partie d'un système de bras en forme de parallélogramme et que le moyen de commande du couple (33,34) de plaques de diaphragme comporte un segment denté (40) et un pignon (42), qui engrène avec le segment denté (40), ce dernier étant monté directement sur un bras (36) du système de bras en forme de parallélogramme.

3. Diaphragme du rayonnement primaire suivant la revendication 1, caractérisé par le fait que les plaques (23,24) de chaque couple de plaques de diaphragme font partie d'un système en forme de parallélogramme articulé ; et que les moyens de commande du couple (23,24) de plaques du diaphragme comportent un segment denté (25) et un pignon (31), qui engrène avec le segment denté (25), le segment denté (25) lui-même formant un bras du système en forme de parallélogramme articulé.

4. Diaphragme du rayonnement primaire suivant l'une des revendications 1 à 3, caractérisé par le fait que les pignons (31,32,42,43) sont montés de façon fixe sur les arbres (18,19).

5. Diaphragme du rayonnement primaire suivant l'une des revendications 1 à 4, caractérisé par le fait que le premier groupe de couples (23,24) de plaques de diaphragme a des dimensions inférieures à celle du second groupe de couples (33,34) de plaques de diaphragme de sorte que le diaphragme (1) du rayonnement primaire possède la forme d'un tronc de cône.

6. Diaphragme du rayonnement primaire suivant l'une des revendications 1 à 5, caractérisé par le fait que les dents des segments dentés (25,40) sont conformées en fonction des angles des arbres (18,19) par rapport à l'axe longitudinal (17) du diaphragme (1).

7. Diaphragme du rayonnement primaire pour un tube à rayons X comportant un premier groupe de couples de plaques de diaphragme, qui sont perpendiculaires l'une à l'autre, les plaques de chaque couple de plaques du diaphragme faisant partie d'un système en forme de parallélogramme articulé et étant réglables en commun, par rapport à l'axe longitudinal du diaphragme, à l'aide respectivement d'un segment denté, chaque segment denté pouvant tourner autour d'un axe sous l'action d'un pignon respectif, caractérisé par le fait que le diaphragme du rayonnement primaire comporte un second groupe de deux couples de plaques de diaphragme, perpendiculaires entre eux, qui correspond, du point de vue agencement et possibilités de réglage, au premier groupe de couples (23,24) de plaques de diaphragme et qui est disposé à distance du premier groupe de couples (23,24) de plaques de diaphragme, lorsqu'on regarde dans la direction longitudinale de l'axe longitudinal (17), le premier groupe de couples (23,24) de plaques de diaphragme et le second groupe de couples (33,34) de plaques de diaphragme étant dirigés de la même manière symétriquement par rapport à l'axe longitudinal (17), tandis qu'un pignon (31) pour le réglage d'un couple (23,24) de plaques de diaphragme du premier groupe et un pignon correspondant (42) pour le réglage d'un couple (33,34) de plaques de diaphragme du second groupe sont montés sur un arbre rotatif commun (18).

8. Diaphragme du rayonnement primaire suivant la revendication 7, caractérisé par le fait que l'autre pignon (32) pour le premier groupe de couples de plaques de diaphragme et l'autre pignon (43) pour le second groupe de couples de plaques de diaphragme sont montés fixes sur un arbre rotatif commun (19).

9. Diaphragme du rayonnement primaire suivant la revendication 7 ou 8, caractérisé par le fait que les segments dentés (40) sont disposés directement sur un bras (36) dans le système en forme de parallélogramme articulé.

10. Diaphragme du rayonnement primaire suivant la revendication 7 ou 8, caractérisé par le fait que les segments dentés (25) forment un bras du système en forme de parallélogramme articulé.

11. Diaphragme du rayonnement primaire suivant l'une des revendications 7 à 10, caractérisé par le fait que le premier groupe de couples (23,24) de plaques de diaphragme possède des dimensions inférieures à celles du second groupe de couples (33,34) de plaques de diaphragme, de sorte que le diaphragme (1) du rayonnement primaire possède la forme d'un tronc de cône.

12. Diaphragme du rayonnement primaire suivant l'une des revendications 7 à 11, caractérisé par le fait que les dents des segments dentés (25,40) sont conformées en fonction des angles des arbres (18,19) par rapport à l'axe longitudinal (17) du diaphragme (1).
